(19) Europäisches Patentamt
European Patent Office
Office européen des brevets

(11) Numéro de publication : **0 478 480 A1**

(12) # DEMANDE DE BREVET EUROPEEN

(21) Numéro de dépôt : **91420345.0**

(22) Date de dépôt : **27.09.91**

(51) Int. Cl.$^5$ : **C07D 207/452,**
C07D 207/456, C08F 226/10,
C08J 5/18, G02F 1/35

(30) Priorité : **28.09.90 FR 9011956**

(43) Date de publication de la demande :
**01.04.92 Bulletin 92/14**

(84) Etats contractants désignés :
**AT BE CH DE DK ES FR GB GR IT LI LU NL SE**

(71) Demandeur : **SOCIETE ANONYME: FLAMEL TECHNOLOGIE**
**33, Avenue Georges Lévy, Parc Club du Moulin à Vent**
**F-69200 Venissieux (FR)**

(72) Inventeur : **Camberlin, Yves**
**68 bis, quai Georges Clémenceau**
**F-69300 Caluire (FR)**
Inventeur : **Mignani, Gérard**
**2, avenue des Frères Lumière**
**F-69008 Lyon (FR)**
Inventeur : **Meyrueix, Rémi**
**Le Bois Rambert,42 Rue Hector Berlioz**
**F-69009 Lyon (FR)**
Inventeur : **Tapolsky, Gilles**
**10, cours Vitton**
**F-69006 Lyon (FR)**

(74) Mandataire : **Ropital-Bonvarlet, Claude**
**Cabinet BEAU DE LOMENIE, 51, avenue Jean-Jaurès**
**F-69007 Lyon (FR)**

(54) **Monomère, polymère et matériau en dérivant, éventuellement orienté, actif en optique non linéaire et leurs procédés d'obtention.**

(57) La présente invention concerne un monomère, polymère et matériau en dérivant, éventuellement orienté, actif en optique non linéaire et leurs procédés d'obtention.
Le monomère est caractérisé par le fait qu'il comporte :
— au moins deux doubles liaisons carbone-carbone, chaque double liaison étant activée, c'est-à-dire dont la polymérisation est facilitée par une fonction électroattractrice ;
— un système non centro symétrique de liaisons pi conjuguées portant à l'une des extrémités au moins un groupe électroattracteur et à l'autre extrémité au moins un groupe électrodonneur.
Application à la fabrication de dispositifs actifs en optique non linéaire.

EP 0 478 480 A1

La présente invention concerne de nouveaux composés chimiques et leurs utilisations pour obtenir des matériaux organiques actifs en optique non linéaire (ONL).

Elle se rapporte plus particulièrement à un matériau organique de haut poids moléculaire présentant une concentration élevée en motifs actifs en optique non linéaire, ou hyperpolarisables.

On connait de nombreux matériaux présentant une activité en optique non linéaire. Parmi ces matériaux, les matériaux organiques sont généralement constitués d'une matrice polymérisable dans laquelle est dissous ou incorporé un composé organique hyperpolarisable. Toutefois, ces matériaux présentent certains inconvénients, principalement dus à la solubilité et compatibilité du composé organique dans la matrice.

En outre, dans ces systèmes appelés également systèmes "Guest/host", il persiste toujours une certaine mobilité du composé hyperpolarisable. Ainsi, l'orientation des molécules imposée par soumission du matériau à un champ électrique, sera perdue au cours du vieillissement provoquant une diminution de l'activité en optique non linéaire dudit matériau.

Il a également été proposé des matériaux organiques polycondensés comprenant des groupements hyperpolarisables greffés sur la chaîne principale du polymère par l'intermédiaire de chaînes hydrocarbonées plus ou moins longues.

Comme dans les systèmes précédents du type "Guest/host", il persiste une mobilité résiduelle des groupements hyperpolarisables conduisant à une diminution de l'activité en optique non linéaire au cours du vieillissement du matériau.

Enfin, il a été proposé des matériaux organiques de poids moléculaire élevé obtenu par polycondensation d'un monomère comprenant des fonctions réactives portées par un groupement hyperpolarisable, avec un monomère à fonctions conjuguées.

Ainsi, si les fonctions réactives sont des fonctions hydroxyles, le monomère conjugué pourra être un diisocyanate.

Dans ces matériaux, le groupement hyperpolarisable fait partie de la chaîne principale ou squelette du polymère. En conséquence, la mobilité résiduelle de ces groupements est significativement diminuée car elle requiert une mobilité de la chaîne du polymère. Cette dernière n'est toutefois pas nulle et l'on observe après l'etape d'orientation sous champ électrique, un lent retour vers le désordre entrainant de ce fait une perte progressive de l'activité en optique non linéaire (quadratique).

Par exemple la demande de brevet japonais n° 88-175837 décrit des composés de ce type contenant des unités répétitives de formules suivantes :

$$\text{-[OCONH - R - NHCOO} \text{-(}CH_2)_m \text{ - L -(}CH_2)_m\text{]-} \qquad (A)$$

$$\text{et -[OCONH - R - NHCOO -(}CH_2)_k\text{]-} \qquad (B)$$

dans lesquelles L représente un groupement hyperpolarisable.

Il convient de rappeler que pour qu'un composé ait une activité ONL quadratique, il faut en outre que le composé ait une bonne aptitude à être orienté au moyen d'un champ électrique et à garder cette orientation. Les matériaux actifs en optique non linéaire sont généralement utilisés sous forme de film dans les dispositifs optiques ou optoélectriques, ces films devant être le plus régulier possible et de faible épaisseur (quelques dizaines de micron, $10^{-2}$ à $10^{-1}$ mm).

En outre les matériaux décrits ci-dessus présentent l'inconvénient majeur d'être faiblement filmogènes et faiblement solubles dans les solvants habituellement utilisés pour réaliser les films tels que, le dichlorométhane, le diméthylformamide (DMF), la N-méthyl pyrolidone (NMP), le chloroforme, la cyclohexanone, la méthylisobutylcétone.

Un des buts de la présente invention est notamment de remédier à ces inconvénients en proposant un matériau organique polymèrique présentant une bonne filmogibilité et une solubilité élevée dans les solvants classiques.

Un autre but de la présente invention est de fournir un polymère susceptible d'apporter un telle propriété à ce materiau.

Un autre but de la présente invention est de fournir un monomère dont la polymèrisation ou la copolymèrisation conduisent à un tel polymère

Ces buts et d'autres qui apparaitront par la suite sont atteints au moyen d'un composé caractérisé par le fait que il comporte :

- au moins deux doubles liaisons carbone-carbone, chaque double liaison étant activée, c'est-à-dire dont la polymerisation est facilitée, par une fonction électroattractrice ;
- un système non centro symétrique de liaisons ($\pi$ ) conjuguées portant à l'une des extrémités au moins un groupe électroattracteur et à l'autre extrémité au moins un groupe électrodonneur.

Ledit système non centro symétrique de liaisons $\pi$ conjuguées correspond aux divers systèmes connus de l'homme de métier pour leur activité en optique non linéaire quadratique. On préfère les systèmes peu polymèrisables dont on trouvera dans la suite de la présente description les familles les plus usuelles et des paradigmes.

La fonction électro attractive peut être un noyau aromatique, un atome de métalloïde avantageusement azote, oxygène ou halogène de préférence chlore ; elle peut également être une fonction carboxyle, une fonction sulfone. Enfin, il convient de souligner qu'il peut y avoir plusieurs fonctions électro attractrices pour activer une double liaison.

Ladite fonction électroattractrice est de préfèrence choisie parmi les fontions dérivées de la fonction carboxylique comme par exemple les fonctions nitrile, ester, amide et avantageusement imide, de préférence cyclique et les fonctions porteuses d'un cation tels que les sels de pyridinium.

Les dites doubles liaisons sont de préférence peu encombrées et ne portent sur lesdits carbones, outre lesdites fonctions électroattractrices, que des hydrogènes ou des substituants peu volumineux comme par exemple un chlore ou un methyle, voire un éthyle.

Chacune des dites doubles liaisons est reliée au système non centro symétrique de liaisons pi ($\pi$ ) conjuguées. Ceci est avantageusement fait au moyen de liens L qui peuvent être soit une liaison simple soit une chaine hydrocarbonée.

Les liens L sont de préférence tels que il n'y ait pas interraction entre ladite fonction électroattractrice et ledit système non centro symétrique de liaisons $\pi$ conjuguées. La longueur des liens n'excède en général pas 10 atomes et est de préférence compris entre 2 et 6 atomes .

Les liens préférés sont avantageusement des aryles ou des alcoyles au sens du dictionnaire de chimie de M. C. DUVAL. Les liens sont par exemples une chaine aliphatique interrompue une ou plusieurs fois éventuellement par un héteroatome de préférence chalcogène ou azote, par exemple polyéthylène diamine ou polyéthylène glycol. Toutefois on préfère des liens aryles ou aralcoyles.

A titre d'exemple de liens préférés on peut citer les radicaux divalents suivants :

– les radicaux cyclohexylènes ; phénylènes ; méthyl-4 phénylène-1,3 ; méthyl-2 phénylène-1,3 ; méthyl-5 phénylène-1,3 ; méthyl-6 phénylène-1,3 ; diéthyl-2,5 méthyl-3 phénylène-1,4 ; et les radicaux de formule :

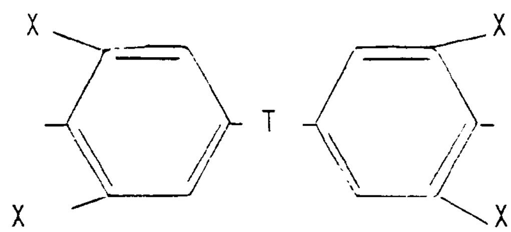

dans laquelle T représente un lien valentiel simple ou un groupement :

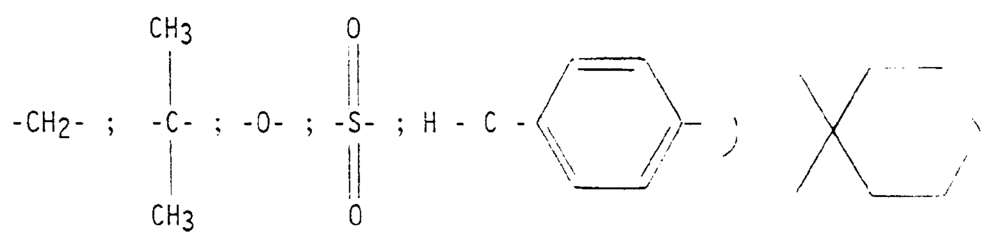

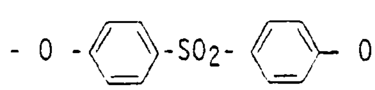

et X représente un atome d'hydrogène, un radical méthyle, éthyle ou isopropyle.

Les points d'attache des dits liens L sur les dites doubles liaisons carbone-carbone, sont choisis de préférence de manière à laisser dégagée la dite double liaison. Ainsi on choisi de préférence un point d'attache du coté de la dite fionction électroattractrice. Par exemple lorsque ladite fonction électroattractrice est choisie parmi les fonctions dérivées de la fonction carboxylique comme par exemple les fonctions ester, amide et avantageusement imide, le lien est attaché à cette fonction sur l'azote ou sur l'oxygène.

Le composé selon la présente invention peut être symbolisé par la formule suivante :

Do-A-Do′

où Do et Do′ différent ou de préférence semblable représentent les dites doubles liaisons activées ;

Où A représente le système à liaison ($\pi$) équipé de deux liens semblables ou différents L et L′ tel que A puisse être représenté par

-L-A′-L′-

avec A′ représentant le système à liaison $\pi$.

De préférence le composé selon la présente invention répond à la formule suivante :

```
Y'                                     Y'
!                                      !
R-C = CH-CO- N (R') A - N (R')- CO - CH = CR          (I)
```

dans laquelle :

les radicaux R représentent indépendamment un atome d'hydrogène ou des radicaux alcoyle ou alcoylidène de bas poids moléculaire (de $C_1$ à $C_6$) éventuellement substitué.

R′ représente indépendamment un groupe acyle de $C_1$ à $C_6$ éventuellement substitué ou un groupe acylidène de $C_1$ à $C_6$, éventuellement substitué et/ou insaturé.

R et R′ étant avantageusement reliés entre eux pour former un hétérocycle de 5 à 7 chainons ;

A est défini ci-dessus.

De préférence les radicaux R et R′ sont choisis de manière que la formule (I) devienne la formule (I′)

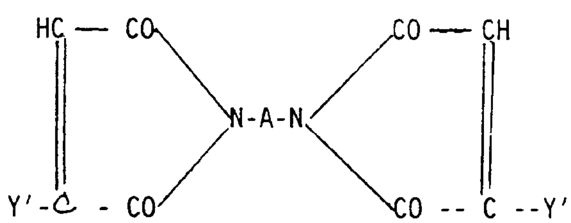

(I′)

dans laquelle :

. les symboles Y′ représentent indépendamment H, $CH_3$ ou Cl ; A′ représente un groupement hyperpolarisable avantageusement de formule:

$$E - Z_1 - X = Y - Z_2 - N-$$

dans laquelle :

– X et Y représentent indépendamment N, C et/ou CH

– $Z_1$, $Z_2$ représentent un groupe aromatique ou insaturé divalent choisi par exemple parmi ceux dérivés le benzène (c'est-à-dire phénylène), la pyridine, le furanne, le styrène, l'acétylène, l'éthylène.

– E représente un groupe électroaccepteur.

Parmi les groupes accepteurs d'électrons, les groupements nitro et cyano sont préférés.

Selon une caractéristique de l'invention, les groupements aromatiques $Z_1$, $Z_2$ qui peuvent porter au moins un substituant R7 sont choisis parmi les groupements phényle, biphényle, naphtyle, les substituants $R_7$ étant choisis indépendamment dans le groupe comprenant les radicaux alkyle inférieur, halogène, amido, amino, sulfoxyde, hydroxy, alcoxy et trifluorométhyle.

Les radicaux préférés sont des radicaux moyennement encombrants, c'est-à-dire qu'ils correspondent à l'encombrement d'un isopropyle ou isoamyle.

Lorsque $R_7$ est situé sur le groupement $Z_1$, il est avantageusement donneur tandis que lorsqu'il est situé sur $Z_2$, il est avantageuse accepteur. Les radicaux $R_7$ sont avantageusement situés en position ortho par rapport à la chaine -X = Y-.

En effet, la présence de ces radicaux en ortho favorise la conformation ou l'isomérie trans laquelle permet l'allongement (ou l'extension) maximale des systèmes non centrosymétriques. Cet allongement maximal est préféré.

Ainsi une des familles préférées des composés selon l'invention est telle que l'enchainement $-Z_1 - X{=}Y - Z_2 -$ réponde à la formule générale :

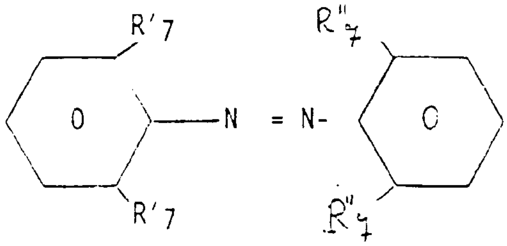

$R'_7$ étant choisi parmi les groupes méthyle, éthyle, isopropyle, propyle, isobutyle et butyle, alcoxy de $C_1$ à $C_4$, amino de $C_1$ à $C_6$. $R''_7$ étant choisi parmi les groupes méthyle, éthyle, isopropyle, propyle, isobutyle, et butyle, trifluorométhyle ou les atomes de chlore ou de fluor voire les groupes amido ou sulfoxyde."

Par radical alkyle inférieur, on entend les radicaux aliphatiques linéaires ou ramifiés comprenant de 1 à 6 atomes de carbone.

Les radicaux $R_7$ préférés de l'invention sont les radicaux méthyle, éthyle et le chlore.

A' peut être tout groupement organique actif en ONL tel que défini et exemplifié dans l'article 2 H.E. KATZ, C.W. DIRK, K.D. Singer, Te. SOHN. Mol. Cryst. Liq. Cryst. Inc. Nonlin. Opt. (1988) 157, 525 et dans les demandes de brevets Français déposées par la demanderesse sous les N° 88 05 214, 88 12 028, 88 12 079, 88 12 080, 89 02 271, 89 04 232, 89 05 870, 89 10 196, 89 10 197, 90 00 377, 90 00 575, 90 02 336, 90 05420.

Un objet supplémentaire de la présente invention est de fournir une technique de synthèses des composés utilisables comme monomère.

Ainsi que cela a été spécifié ci-dessus, le point d'ancrage du lien sur ledit système de liaison a peu d'importance mais il est préférable d'utiliser des fonctions ou des radicaux déjà présents sur lesdits systèmes de liaison . C'est pourquoi les groupements anilines ou alcoxy sont assez pratiques pour l'accrochage.

Ainsi quand la synthèse à liaison $\pi$ possède une fonction aniline, laquelle joue en général le rôle d'un groupe donneur, le point d'ancrage peut alors être l'atome d'azote, comme il est plus facile de faire réagir les fonctions alcool ou de greffe de groupes alcoyles présentant une fonction alcool. Ainsi le remplacement d'un groupement diméthyl amino par un groupe di hydroxyalcoylamino permet de donner un point d'ancrage facile. Lorsque le système à liaison $\pi$ possède un groupement alcoxyphénol, il est facile de remplacer le groupement alcoyl par un groupement mono ou poly hydroxyalcoyle. On peut ainsi remplacer le groupe méthyle par groupement $\omega$ $\omega$ di hydroxy alcoyle. Lorsque ledit système à liaison $\pi$ possède deux groupements alcoxy phénol, il suffit de remplacer chacun des alcoyles par des liens respectivement L et L'.

Comme le groupement donneur préféré est en général un groupement diméthyl amino, on remplace ce dernier par un groupement di hydroxy alcoylamino, ce qui permet les réactions des fonctions alcooliques suivantes : réactions qui, connues en elles-mêmes, permettent d'accéder aux molécules selon la présente invention.

Cette liste de réactions n'est pas exhaustive.

La synthèse des monomères selon l'invention est réalisable selon les techniques chimiques usuelles maitrisées par l'homme de métier. Les voies les plus utiles sont mentionnées ci-après.

Greffage d'une fonction maléimide :

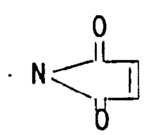

La fonction maléimide sera ci-après symbolisé par - **Mal**.

en général :

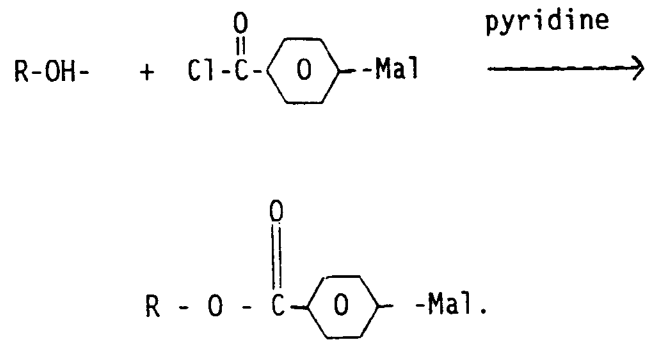


Réaction très classique : on piège HCl par une base, par exemple la Pyridine. Pour plus de détails confère par exemple : RAO et Coll. Journal of Polymers. (1989), 2510. C'est le mode opératoire le plus simple. La réaction marche mutatis mutandis pour des liens non aromatiques :

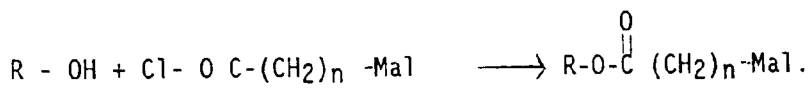

Synthèse de 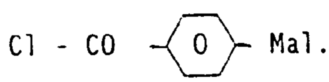

(même référence) On peut également envisager la séquence

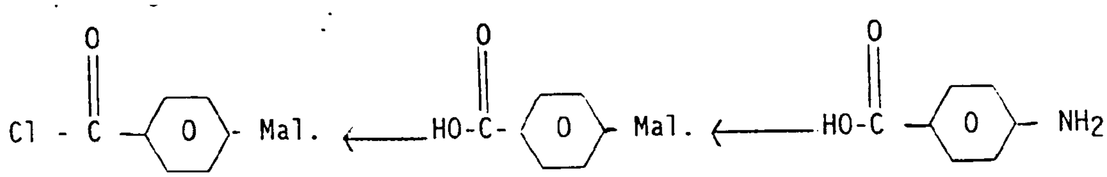

Greffages d'autres doubles liaisons activées

<u>Double liaisons activées par un noyau aromatique (type styrène)</u>

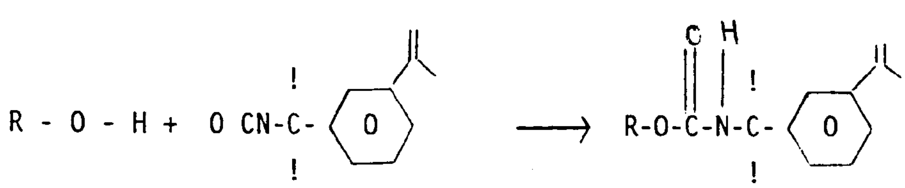

Produit vendu par American cyanamide (TMI)

Réaction alcool + isocyanate → uréthanes (carbamates) Voire : SATCHELL et SATCHELL, Chem. Soc. Rev., 4, (1975), 231.

Autres méthodes possibles :

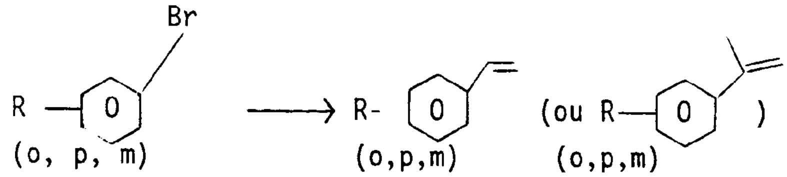

Réaction de HECK : catalyse Palladium 0

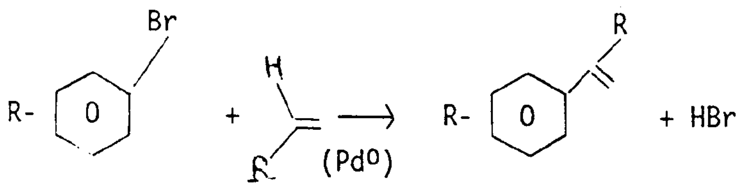


Pour la réaction de HECK
HECK, Org. React., 27 (1982), 345.
HECK, Ado. Catal., 26 (1977), 323.
JIRA, Organometal. React., 3, C (1972), 1-190.

Doubles liaisons activées par un silicium Si Vinyl :

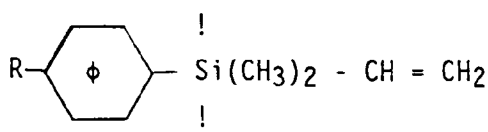

Méthode par utilisation des Grignards

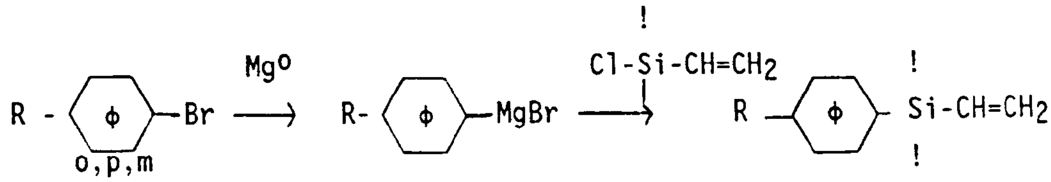


par exemple :

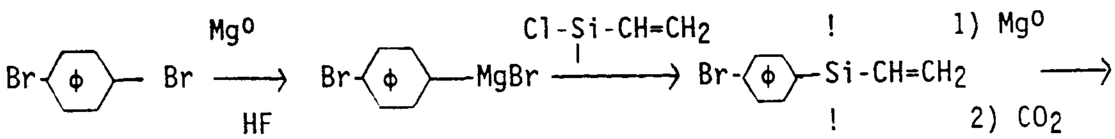


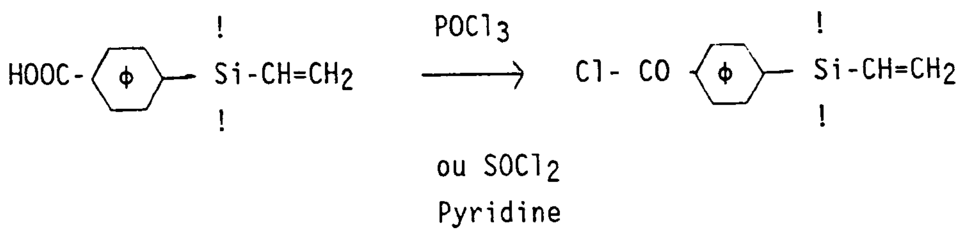


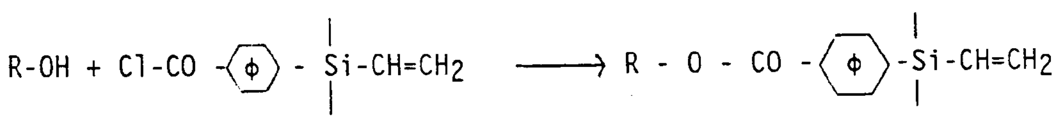

Les réactions de type GRIGNARD :

"The chemistry of the carbonyl group". PATAI ;
GRIGNARD Reactions of Non metallic substances.
KHARASCH et REINMUTH.
Prentice-Hall, Englewood Cliffs, 1954.
Doubles liaisons activées par un azote par exemple :

Enamines :

en général

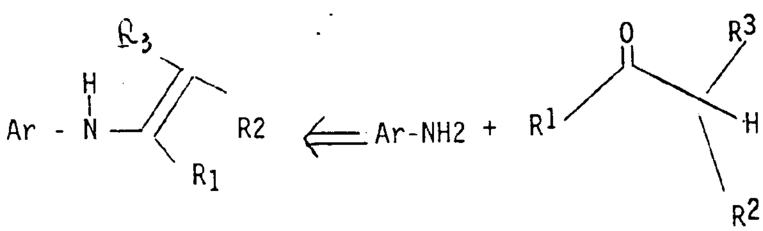

Référence : SZMUSZKOVICZ :
Adv. Org. Chem., 4, (1963), 1-113.

Doubles liaisons activées par un oxygène tel que Enols :

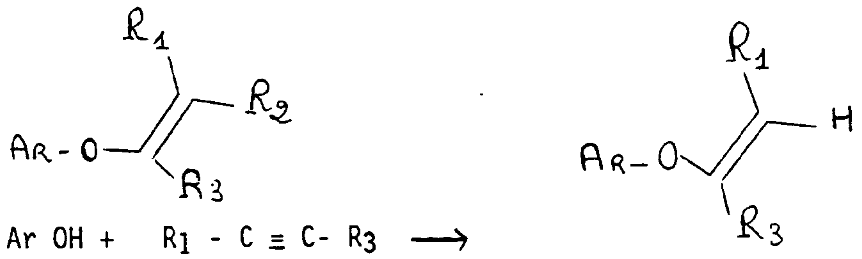

Référence : Advanced Organic Chemistry
Third Edition : Jerry March.
(1985) J. Wiley and Sons.

1 Accès aux précurseurs organosolubles

Dans notre cas, tous les colorants connus sous le terme de Red doivent posséder des fonctions "diols" de type :

$$HO - (CH_2)_n - N - (CH_2)_n\ OH$$

en général ; le plus industriel :

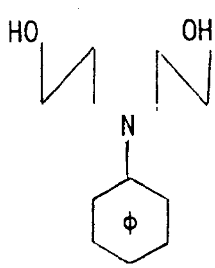

par exemple :

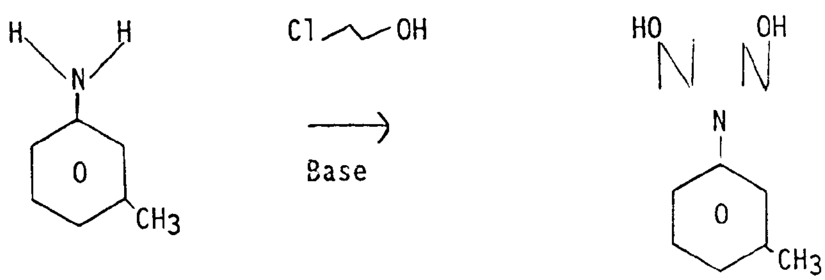


voire par exemple : mode opératoire
W - ROSS ; J. Chem.Soc., (1949), 183.

2 Accès aux "Red" de type diazo

On utilise en général une réaction de copulation classique, par exemple

Synthèse du Red 17.

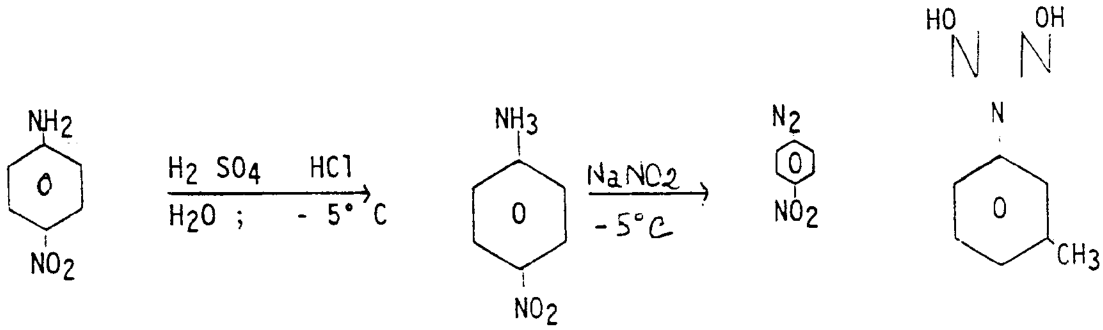


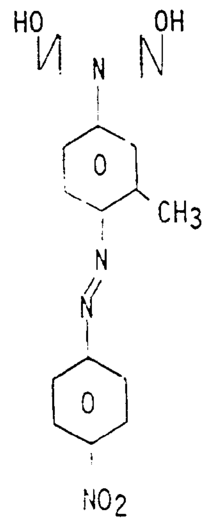

Red 17

Mode opératoire général

On introduit dans un réacteur une solution diluée d'acide fort (type HCl ou $H_2SO_4$) ; 50 % W ; on rajoute ensuite un équivalent d'aniline (par exemple :

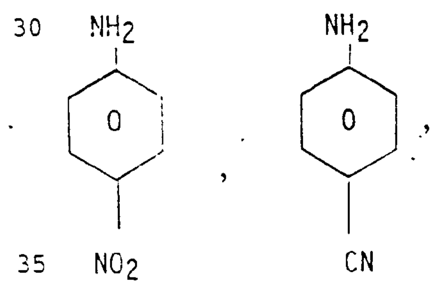

, ....) ;

après dissolution totale on refroidit vers - 5°C (à l'aide d'eau glacé) et on rajoute ensuite un équivalent de $NaN0_2$ dans le minimum d'eau. On laisse le diazo se faire ( 1/2 h à 1 heure). On rajoute ensuite le composé à copuler (en général

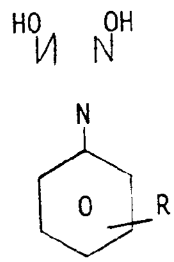

On laisse réagir 1 heure à 2 heures à température ambiante. Il y a formation d'un précipité ; on amène ensuite le PH vers 3 à l'aide d'une addition de $CH_3C0_2Na$ ; on filtre ; on lave à l'eau (PH = 7) ; on sèche sous vide ; on recristallise si nécessaire (en général dans l'alcool) ; on récupère le diazo avec un rendement de l'ordre de 70-95 %.

La purification de red s'effectue à l'aide d'une recristallisation (éthanol, méthanlol,....).
par exemple :
"AZO and diazo chemistry" : ZOLLINGER. Interscience publishers Inc ; New York, 1961.

3 Accès aux Red de type stilbène

Par exemple dans le brevet US 4757130 accès au stilbène suivant :

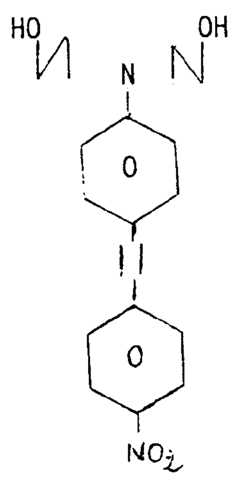

1

Un composé de type 1 peut être préparé via une réaction de condensation

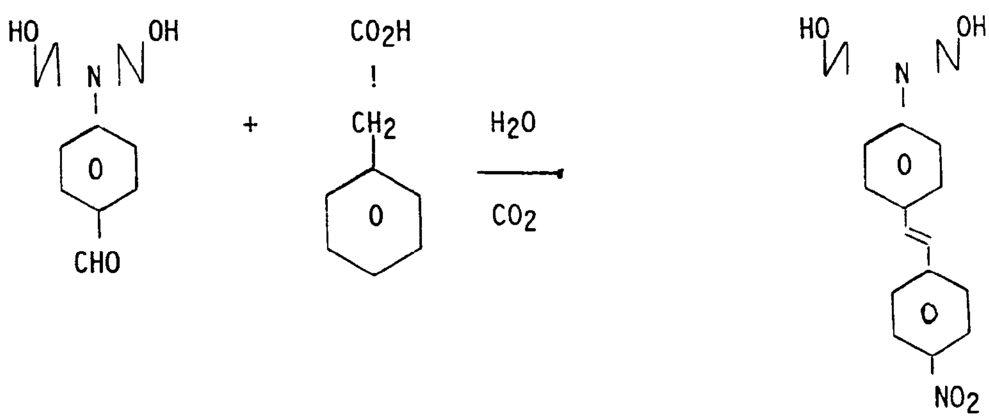


voire en particulier : thème sur la synthèse des stilbène.
K. BECKER ; Synthèse, 341 (1983).

4 Accès aux Red. de type alcyne par exemple

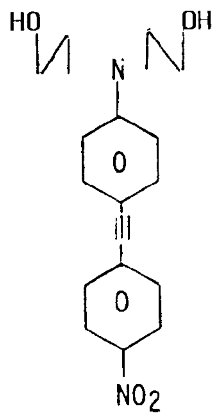

(référence US4603187)

pour la synthèse : Organic Synthesis.
Vol. III, 786 (1955)
Vol. IV, 857 (1963)

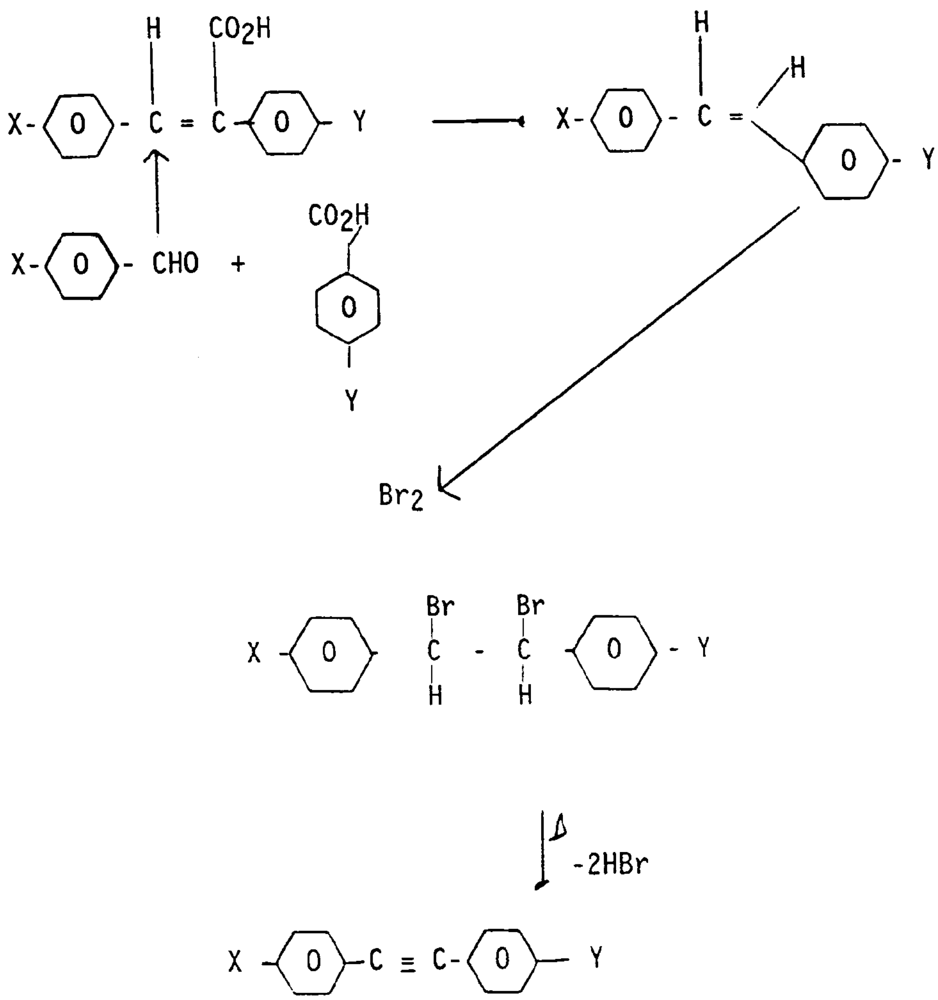


Un objet supplémentaire de la présente invention est de fournir des polymères fait à partir des composés selon la présente invention

Une des surprises majeures de l'invention réside dans la remarquable stabilité du système de liaison $\pi$ dans les conditions de polymérisation en présence de doubles liaisons activées qui polymérisent elles très facilement. Il est ainsi possible de prévoir une très large palette de polymères selon la présente invention dont la

caractéristique sera de contenir au moins un motif dérivé des composés monomères visés par la présente invention.

La formation des (co) polymères selon la présente invention comporte au moins 10 % avantageusement au moins 1/3 en mole de monomère(s) actif(s) en O.N.L..

Elle peut comporter en outre divers monomères courants non actifs en O.N.L.

Comme formulation donnant de bons résultats, il convient de mentionner une formulation comportant :

– (a) un ou plusieurs N, N'-Bis-imide (s) de formule :

Y' Y'

! !

R-C = CH-CO- N (R') $A_1$- N (R')- CO - CH = CR (I")

dans laquelle :

les radicaux R représentent indépendamment un atome d'hydrogène ou des radicaux alcoyle ou alcoylidène de bas poids moléculaire (de $C_1$ à $C_6$) éventuellement substitué.

R' représente indépendamment un groupe acyle de $C_1$ à $C_{10}$ éventuellement substitué ou un groupe acylidène de $C_1$ à $C_6$, éventuellement substitué et/ou insaturé.

R et R' étant avantageusement reliés entre eux pour former un hétérocycle de 5 à 7 chainons ;

$A_1$ représente A défini ci dessus ou A" défini ci-dessous.

De préférence les radicaux R et R' sont choisis de manière que la formule (I) devienne la formule (I')

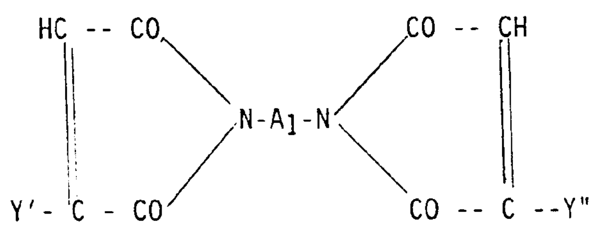

(I')

dans laquelle :

. les symboles Y' et Y" représentent indépendamment H, $CH_3$ ou Cl ;

. le symbole A" représente un radical divalent de préférence choisi dans le groupe constitué par les radicaux : cyclohexylènes ; phénylènes ; méthyl-4 phénylène-1,3 ; méthyl-2 phénylène-1,3 ; méthyl-5 phénylène-1,3 ; méthyl-6 phénylène-1,3 ; diéthyl-2,5 méthyl-3 phénylène-1,4 ; et les radicaux de formule :

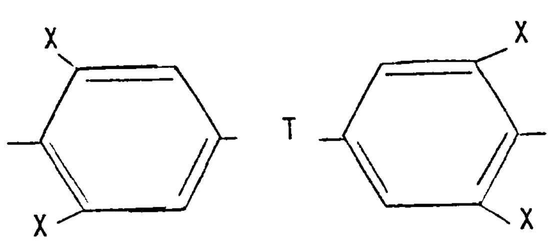

dans laquelle T représente un lien valentiel simple ou un groupement :

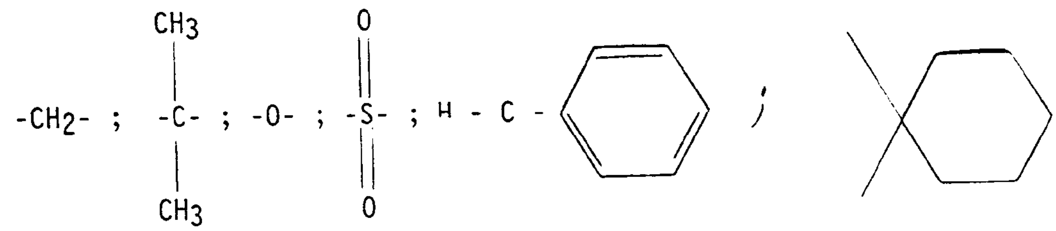

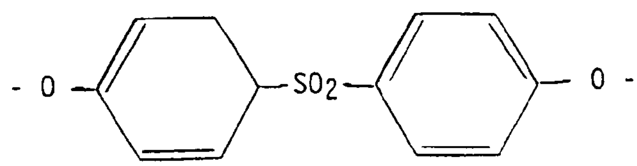

et X représente un atome d'hydrogène, un radical méthyle, éthyle ou isopropyle ;

– un (ou des) réactif(s) ( b ) et/ou ( c ) ;

( b ) consistant dans un ou plusieurs hétérocyle(s) substitué(s) notamment par des chaines courtes ($C_2$-$C_6$) insaturées tels que : les vinylpyridines, la N-vinylpyrrolidone-2, l'isocyanurate d'allyle, le vinyltétrahydrofuranne ;

( c ) consistant dans un composé du type styrène, tel que par exemple 1' $\alpha$ -méthyl-styrène, le divinyl benzène ou l'éthyl-vinyl-benzène. Lorsque l'on désire que le réactif b ou c soit actif en ONL lesdits hétérocycles substitués ou composés du type styrène joue le rôle de doubles liaisons activées.

Le rapport $\frac{b + c}{a}$ pouvant varier de 1/20 à 10 de préférence de 1/10 à 1.

– et le cas échéant pour améliorer la rhéologie un ou plusieurs réactifs acrylate (d) consistant dans un ou plusieurs composé(s) de formule générale :

$$(CH_2 = CR_6 - CO - O )_n \text{———} B \qquad (II)$$

dans laquelle :

. le symbole $R_6$ représente un atome d'hydrogène ou un radical méthyle;

. n représente un nombre entier ou fractionnaire au moins égal à 1 et au plus égal à 8 ;

. le symbole B représente un radical organique de valence n dérivé : d'un reste aliphatique saturé, linéaire ou ramifié, ayant de 1 à 30 atomes de carbone et pouvant renfermer un ou plusieurs pont(s) oxygène et/ou une ou plusieurs fonction(s) hydroxyle(s) libre(s) ; d'un reste aromatique (de type arylique ou arylaliphatique) ayant de 6 à 150 atomes de carbone constitué par un noyau benzénique, pouvant être substitué par un ou trois radicaux alkyles ayant de 1 à 5 atomes de carbone, ou par plusieurs noyaux benzéniques, éventuellement substitués comme indiqué ci-avant, reliés entre eux par un lien valentiel simple, un groupement inerte ou un radical alkylène ayant de 1 à 3 atomes de carbone, ledit reste aromatique pouvant renfermer à divers endroits de sa structure un ou plusieurs pont(s) oxygène et/ou une ou plusieurs fonction(s) hydroxyle(s) libre(s), le (ou les) valence(s) libre(s) du radical B aromatique pouvant être portée(s) par un atome de carbone d'une chaine aliphatique et/ou par un atome de carbone d'un noyau benzénique. Le symbol B peut également présenter un radical A.

La quantité de réactif étant telle que le rapport massique d/a peut varier de 0 à 1/10.

Optionnellement :

e). un ou plusieurs monomères de formule III, V, V :

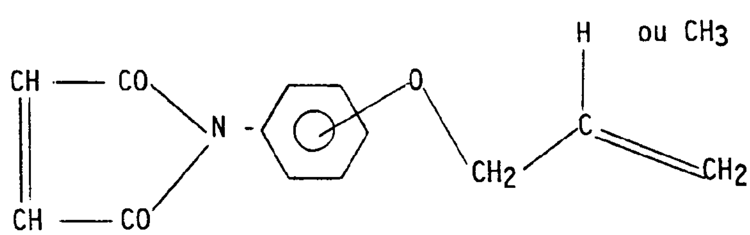 (III)

dans laquelle le radical allyloxy ou méthallyloxy est en position ortho, métha ou para par rapport à l'atome de

carbone du cycle benzénique relié à l'azote ;

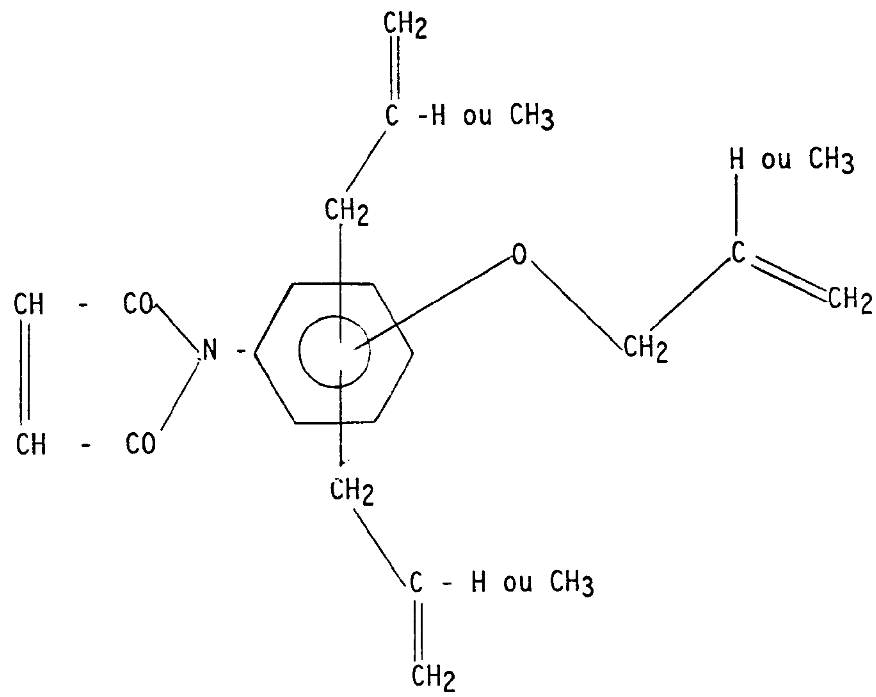

(IV)

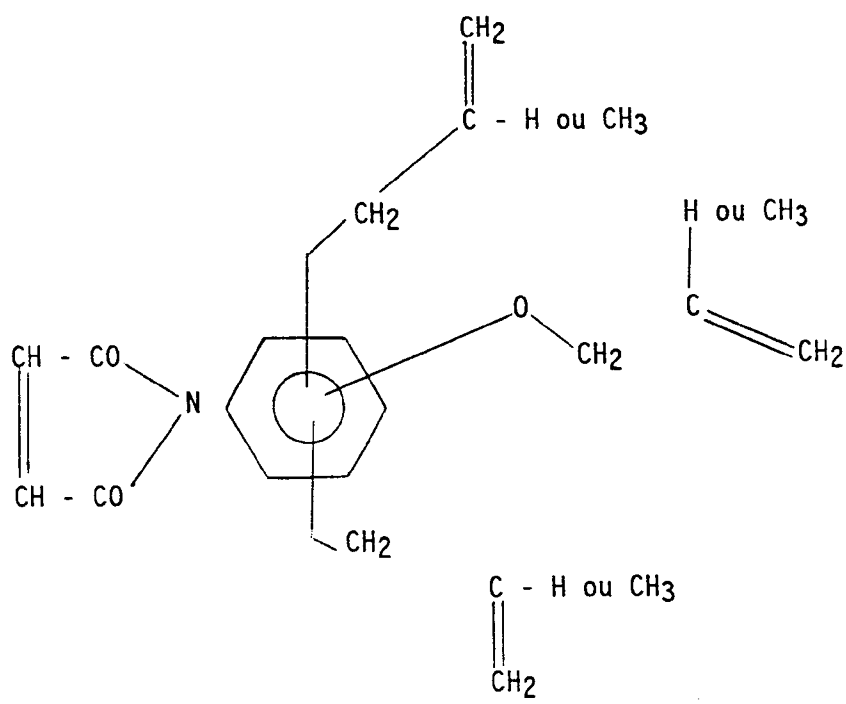

(V)

Dans le composé précité, les proportions des divers constituants du mélange des produits de formules (III) (IV), et éventuellement (V) peuvent varier dans de larges limites.

Chacune des espèces a b c d ou e peut être active en ONL, la contrainte étant que au moins 5 % avantageusement au moins 10 %, de préférence au moins 1/4 en mole des motifs totaux est actif en ONL.

A titre d'exemples spécifiques de bis-imides non actifs en ONL (a) de formule (I"), on peut citer en particulier les composés indiqués dans la demande française 86/17918, c'est à dire :

– le N, N'-métaphénylène-bis-maléimide,
– le N, N'-paraphénylène-bis-maléimide,
– le N, N'-4,4' -diphénylméthane-bis-maléimide,
– le N, N'-4,4' -diphényléther-bis-maléimide,

– le N, N-4,4' - diphénylsulphone-bis-maléimide,
– le N, N'-cyclohexylène-1,4-bis-maléimide,
– le N, N'-4,4'-diphényl-1,1 cyclohexane-bis-maléimide,
– le N, N' -4,4' -diphényl-2,2 propane-bis-maléimide,
– le N, N' -4,4' -triphénylméthane-bis-maléimide,
– le N, N' -méthyl-2 phénylène-1,3-bis-maléimide,
– le N, N' -méthyl-4 phénylène-1,3-bis-maléimide,
– le N, N' -méthyl-5 phénylène-1,3-bis-maléimide.

Ces bis-maléimides peuvent être préparés selon les procédés décrits dans le brevet américain US-A-3.018.290 et le brevet Anglais GB-A-1.137.290. On utilise de préférence, pour la mise en oeuvre de la présente invention, le N, N'-4,4' -diphénylméthane-bis-maléimide pris seul ou en mélange avec le N, N' -méthyl-2 phénylène-1,3-bis-maléimide, le N, N' -méthyl-4 phénylène-1,3-bis-maléimide et/ou le N, N'-méthyl-5 phénylène-1, 3-bis-maléimide.

A titre de réactif acrylate (d) qui convient, on peut citer :

(d1) les mono(méth)acrylates correspondant à la formule (II) dans laquelle :
. n = 1, et
. B représente un radical organique monovalent de formule :

$$-(CH_2\ CH_2O)_m —— B_1 \qquad (VI)$$

dans laquelle : $B_1$ représente un radical alkyle, linéaire ou ramifié, ayant de 1 à 6 atomes de carbone ou un radical phényle ;
et m est un nombre entier égal à zéro, 1 à 9 ;

(d2) les di(méth)acrylates correspondant à la formule (IV) dans laquelle :
. n = 2, et
. B représente un radical organique divalent de formule :

$$—(CH_2\ CH_2O)_p —— B_2 ———— (OCH_2\ CH_2)_q \qquad (VII)$$

dans laquelle: $B_2$ représente un radical divalent alkylène, linéaire ou ramifié, ayant de 2 à 9 atomes de carbone et pouvant renfermer un ou plusieurs pont(s) oxygène ou un radical de formule

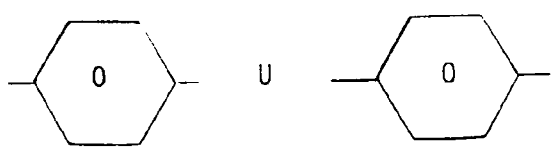

dans laquelle le symbole U représente un lien valentiel simple ou un groupement :

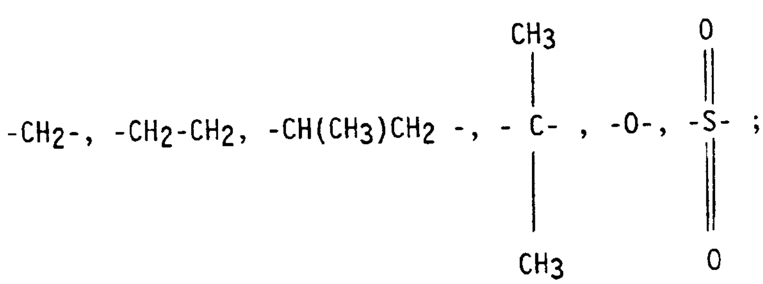

les symboles p et q, identiques ou différents, représentent chacun un nombre entier égal à zéro, 1, 2, 3, 4 ou 5 ;

(d3) les tri- et tétra(méth) acrylates correspondant à la formule (VI) dans laquelle :
. n = 3 ou 4, et
. B représente un radical organique trivalent ou tétravalent dérivé d'un reste aliphatique saturé, linéaire ou ramifié, ayant de 3 à 20 atomes de carbone et pouvant renfermer un ou plusieurs pont(s) oxygène et/ou

une ou plusieurs fonction(s) hydroxyle(s) libre(s) ; (d4) les (méth)acrylates d'époxy novolaques qui, tout en correspondant à la formule (IV), sont représentés içi par la formule suivante :

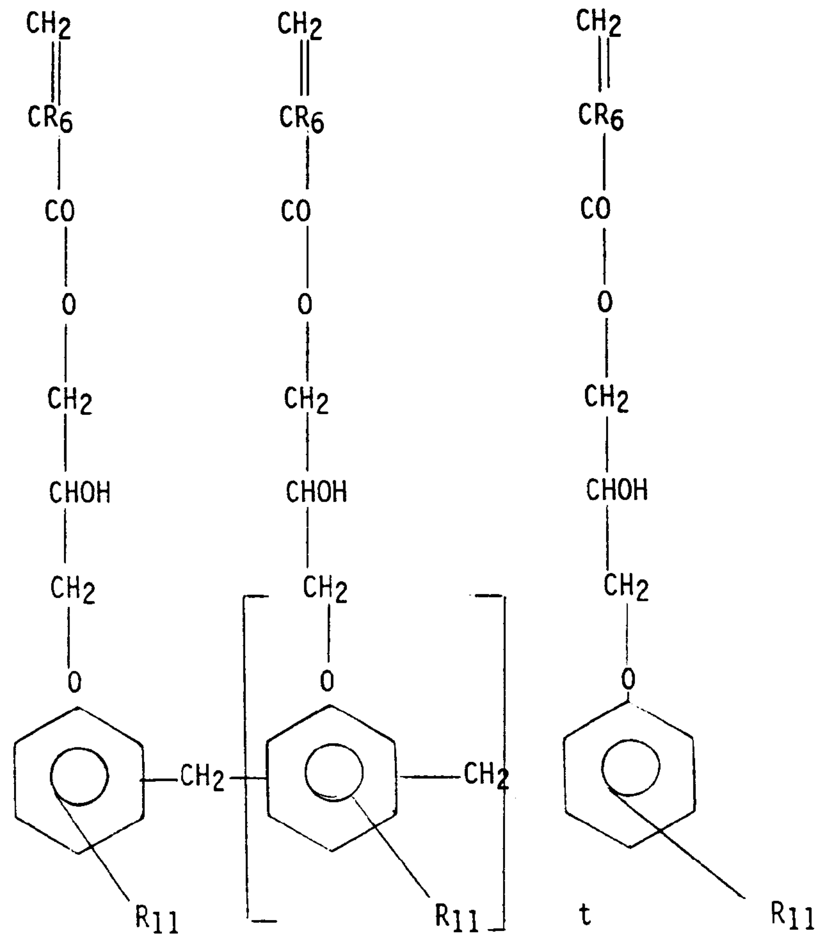

(VIII)

dans laquelle :

. le symbole $R_6$ a la signification donnée ci-avant à propos de la formule (II) ;

. le symbole $R_{11}$ représente un atome d'hydrogène ou un radical méthyle;

. t est un nombre entier ou fractionnaire se situant dans l'intervalle allant de 0,1 à 7 ;

(d5) des mélanges de plusieurs acrylates et/ou méthacrylates d'un même type [ (d1), (d2), (d3) ou (d4) ] entre eux ou des mélanges d'un ou plusieurs acrylate(s) et/ou méthacrylate(s) d'un même type avec un ou plusieurs acrylate(s) et/ou méthacrylate(s) d'un autre type, les composants a,b, c ou d, peuvent être actifs ou non actifs en ONL mais la quantité de composant actif en ONL total est au moins égal à 10 % molaire.

A titre d'exemples spécifiques de réactif acrylate (d1) qui conviennent bien, on peut citer en particulier : les mono(méth)acrylates de méthyle ; les mono(méth)acrylates de phénol(mono-oxyéthylé) ; les mono(méth)acrylates de phénol (di-oxyéthylé).

A titre d'exemples spécifiques de réactif acrylate (d2) qui conviennent bien, on peut citer : les di(Meth)acrylates d'ethyleneglycol ; les di(méth)acrylates de butanediol-1,4 ; les di(méth)acrylates d'hexanediaol-1,6 ; les di(méth)acrylates de tripropylèneglycol ; les di(méth) acrylates des diphénols, di(mono-ou poly-oxyéthylés) ou non , suivants : le dihydroxy-4,4' -diphénylméthane, le bisphénol A, le dihydrocy-4,4' -diphényléther et en particulier les di(méth)acrylates de bisphénol A di(mono-oxyéthylé) ou les di(méth)acrylates de bisphénol A di (di-oxyéthylé) [ cf. formule (VII) dans laquelle $B_2$ représente le radical:

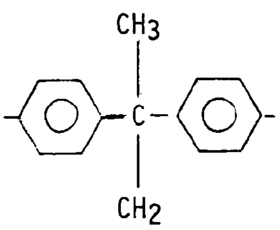

et p = q = 1 ou 2 ]

A titre d'exemples spécifiques de réactif acrylate (d3) qui conviennent bien, on peut citer en particulier : les tri(méth)acrylates de butanetriol-1,2,4 ; les tri(méth)acrylates d'hexanetriol-1,2,6 ; les tri(méth)acrylates de triméthylolpropane ; les tri(méth)acrylates de pentaérythritol ; les tétra(méth)acrylates de pentaérythritol.

Les (méth) acrylates d'époxy novolaques (d4) sont des produits connus dont certains sont disponibles dans le commerce. Ils peuvent être préparés en faisant réagir les acides (méth)acryliques avec une résine époxy de type novolaque, cette dernière étant le produit de réaction d'épichlorhydrine et de polycondensats phéno/formol dans la formule (VIII) donnée ci-avant $R_{11}$ est alors une atome d'hydrogène ou de polycondensats crésol/formol dans la formule $R_{11}$ est alois un radical méthyle. Ces polyacrylates oligomères (d4) et un procédé pour les préparer se trouvent décrits par exemple dans le brevet américain US-A-3.535.403.

A titre d'exemples spécifiques de réactif acrylate (d4) qui conviennent bien, on peut citer en particulier les acrylates d'époxy novolaques de formule (VIII) dans laquelle $R_6$ et $R_{11}$ représentent un atome d'hydrogène et t est un nombre entier ou fractionnaire se situant dans l'intervalle allant de 0,1 à 5.

A titre d'exemples spécifiques de réactif acrylate (d5) qui conviennent bien, on peut citer les mélanges des (méth)acrylates d'époxy novolaques (d4) avec au plus 30 % en poids, par rapport au poids du mélange (d4) + (d3), d'un triacrylate et/ou d'un triméthacrylate répondant aux définitions données ci-avant à propos du réactif acrylate (d3) et en particulier les mélanges des acrylates d'époxy novolaques convenant bien, juste cités ci-avant, avec au plus 25 % en poids, par rapport au poids du mélange, d'un triacrylate et/ou d'un triméthacrylate convenant bien choisi parmi ceux juste cités ci-avant.

Le réactif acrylate (d) qui est utilisé de manière très préférentielle est pris dans le groupe formé par : les di(méth)acrylates de bisphénol A di(mono-oxyéthylé) ; les di(méth)acrylates d'époxy novolaques de formule (VIII) dans laquelle $R_6$ et $R_{11}$ représentent un atome d'hydrogène et t est un nombre entier ou fractionnaire se situant dans l'intervalle allant de 0,1 à 5, ces composés étant pris seuls ou en mélange avec au plus 25 % en poids, par rapport au poids du mélange, de triacrylate de triméthylolpropane.

Le réactif acrylate (e), formé d'un ou plusieurs composé(s) de formule (IV), est utilisé en quantités représentant généralement de 1 % à 60 % et, de préférence, de 5 % à 30 % du poids total des réactifs (a) + (b) + éventuellement (c) + (d).

Avantageusement, les polymères ci-dessus présentant une viscosité à l'état fondu mesurée dans les conditions définies ci-après comprise entre 0,1 Pa.s et 500 Pa.s.

La polymérisation peut être réalisée par toute technique connue de l'homme de métier, notamment par voie thermique ou par faisceau d'électrons. Lorsque l'on désire obtenir des films ou tout produit phylliteux, il est préférable de faire une prépolymérisation pour obtenir des polymères dont le nombre moyen de monomères est compris entre environ 5 et environ 100 (dans la présente description les zéros de position ne sont pas des chiffres significatifs).

Cette prépolymérisation qui permet d'obtenir un collodion, est avantageusement réalisée par voie thermique de préférence à une température comprise entre 50 et 200°C, selon les techniques connues de l'homme de métier.

Une fois la mise en forme et l'éventuelle polarisation réalisées, la polymérisation est poursuivie avantageusement à froid, par irradiation de préférence sous faisceau d'électrons (avec une dose entre 1 et 50 M. Rad (10-2 grades) par µm d'épaisseur sous $1.10^{21}$ électrons volts/s). Un mode opératoire est détaillée dans la suite de la présente description.

Le matériau de l'invention peut être utilisé sous des formes quelconques telles que films, pièces moulées, extrudés, etc..

Il est également possible, sans pour cela sortir du cadre de l'invention, d'ajouter différents composants ou additifs dans le matériau pour améliorer ces propriétés mécaniques ou de mise en forme, par exemple des ignufigeants, des antistatiques, antioxydants, stabilisants ou analogues.

L'invention a également pour objet un dispositif optique ou optoelectrique comprenant au moins un élément constitué par le matériau selon l'invention.

Un autre objet est de fournir un collodion filmable et orientable

Les polymères selon la présente invention ont montré de grandes dispositions à être polarisés et à maintenir cette polarisation au cours du temps.

Les techniques de polarisation telles que celles exposées dans la présente description permet d'obtenir une valeur moyenne de cos θ (<cos θ >) est en général compris entre $10^{-2}$ et 0,5, plus généralement entre 0,1 et 0,2. Une tension de 20 à 25 v/μm donne environ un cos θ de 0,1. Comme l'on se place dans la zone où le cos θ croît linéairement en fonction du champ, il est possible en augmentant la différence de potentiel entre les deux faces du film, d'augmenter l'orientation du polymère en augmentant le champ jusqu'à une valeur d'environ 100 volts/μ m zone à laquelle il y a inflexion, l'orientation cessant de croître proportionnellement avec le champ. Rappelons que θ est l'angle formé par l'orientation des dipôles avec le champ électrique.

Cette polarisation ne décroît que très lentement au cours de temps au contraire des autres matériaux utilisés dans l'optique non linéaire. Ceci doit être comparé avec le composé constitué de red. 17 (10 %) et de polymétacrylates de méthyle (environ 90 %) qui a été étudié dans l'article de M. A. Montazani et al. J. Opt. Soc. Am. (B) vol.6 N° 4 page 733 (1989) dans lequel il était indiqué qu'en un mois 50 % de la polarisation avait disparue.

Par ailleurs, la présence des substituants dans les chaînes aromatiques $Z_1$, $Z_2$ permet d'obtenir des matériaux présentant une longueur d'onde maximale d'adsorption (λ max) au moins équivalente, et généralement supérieure à celle des matériaux ne comprenant pas de substituants.

Pour l'opération de la mise sous forme de film et de polarisation, on utilise en général des techniques décrites sommairement ci-après.

Mise sous forme de film

Sur une plaque de verre recouverte d'une couche conductrice transparente à la longueur d'onde qui sera utilisée, le collodion dont la viscosité est ajustée à une valeur comprise entre $1/2.10^{-2}$ et $1/4.10^3$, de préférence de 50 à 200 centipoises (5 à 20 Pa.s) par addition ou évaporation de solvants et filmé à "la barre de Mayer" et ce en utilisant un appareil tire film tel que par exemple celui vendu sous la dénomination commerciale K control coater de marque Erichsen motorisé. Le film ainsi obtenu est séché progressivement jusqu'à une température au plus voisine de la température de transition vitreuse ($t_g$) ; le film est alors séché sous vide à une température au plus voisine de celle de la transition vitreuse pendant 24 Heures. Une fois le film sec, on dépose une seconde couche conductrice et deux électrodes sont déposées sur les couches conductrices. Les couches conductrices sont soit en or, soit en divers oxydes conducteurs, tel que l'oxyde d'étain ou les oxydes mixtes d'étain et d'indium. L'épaisseur des films selon la présente invention varie en général entre 0,1 et 100 μm, avantageusement entre 1 et 50, de préférence entre 1 et 20μm.

Polarisation

La polarisation est effectuée à une température au plus égale à la température de transition vitreuse finissante de préférence voisine de la température de transition vitreuse commençante. La tension de polarisation est appliquée entre les deux couches d'or. Cette tension est choisie entre 1 et 200 volts par micro/mètre (μ m ). Il est toutefois difficile et rare d'obtenir cette dernière valeur car les films ont tendance à claquer sous une forte différence de potentiel, c'est la raison pour laquelle on utilise plus couramment une tension comprise entre 10 et 100 volts par micro mètre, plus généralement encore entre 20 et 30 v/μ m.
Lorsque la polarisation ne varie plus significativement au cours du temps, on arrête alors l'opération en refroidissant le film en maintenant une tension de polarisation. Cette tension de polarisation peut être légèrement inférieure à la tension de polarisation initiale.

De manière surprenante, il a été montré au cours de l'étude qui a conduit à la présente invention, qu'il est possible de réticuler à chaud en préservant la polarisation. Ce phénomène est extrêmement surprenant car d'une part la température augmente, la conductivité électrique et limite ainsi la faculté à supporter un champs intense et d'autre part le désordre créé par la température s'oppose à l'ordre induit par le champs. Or selon la présente invention, on a pu montrer que les polymères visés par le présent titre pouvaient être polarisés avec thermoréticulation sans que cela impliquât une perte importante de <cos θ>.
Selon une mise en oeuvre préférée de polarisation conjointe à la thermoréticulation, le film, comme précédemment, est soumis à un champs électrique à une température voisine de la température de transition vitreuse finissante jusqu'au moment où l'orientation cesse de croître. Une fois cette valeur atteinte, on maintient la température ou on l'élève au dessus de la température de transition vitreuse finissante d'une valeur au plus égale à 20°C, avantageusement 10°C de préférence 5°C. On constate alors que la température de transition vitreuse s'élève également ce qui permet d'augmenter la température à laquelle est soumis le film. On poursuit l'opération jusqu'à obtenir la température de transition vitreuse désirée généralement de 200°C. La perte de la polarisation au cours du temps est remarquablement réduite.

Ces matériaux sont notamment utilisés dans les dispositifs optiques et optoélectriques tels que, par exemple, des interrupteurs, modulateurs, coupleurs directionnels, bistables.

Les avantages détails et buts de l'invention apparaîtront plus clairement au vu des exemples donnés ci-dessous uniquement à titre indicatif.

La présence des substituants modifie la structure électronique de la molécule. Ainsi, il est possible de régler et modifier la longueur d'onde d'absorption maximale (λ max) du polymère. A titre d'exemple, les substituants électrodonneurs tels que les radicaux alkyles portés par le radical $Z_1$ permettent d'augmenter le λ max, au contraire les substituants électroaccepteurs tels que les halogènes portés par le radical $Z_1$ diminuent le λ max (voir l'article publié par J.B.Dickey et Al dans Am. Dyestuff reporter (1965) page 44).

Lorsque A′ correspond à la formule $E-Z_1-X=Y-Z_2N$, la présence d'un substituant en position ortho par rapport au groupement -X=Y-, porté par le radical $Z_1$ et le radical $Z_2$ favorise la conformation trans de la molécule. Cette conformation permet une meilleure délocalisation électronique dans la molécule et donc augmente l'hyperpolarisabilité de celle-ci (voir l'article S.Yamahamoto et col, bull. Chem. Soc. Jap.44 (1971) 2018).

Exemple 1

**Synthèse d'un monomère pour l'élaboration de polymères de type bis maléimide en application ONL.**

Ce monomère est obtenu selon le mode opératoire suivant :

Dans 300 ml de diméthylacétamide, on dissout, sous atmosphère inerte içi sous azote, 15 g (64 mmol) de chlorure d'acide p-maléimide-benzoïque (2) avec 1 % de NaI comme catalyseur puis l'on ajoute 5 g de (3) (16,4 mmol) et 34 mmol de tributylamine. Le mélange est agité et chauffé à 40-45°C pendant environ 12 h. La solution est alors filtrée puis précipitée dans 1 000 ml d'eau. Le solide est lavé à l'eau puis séché sous vide pendant une douzaine d'heures à température ambiante. Il est chromatographié sur silice avec comme éluant le dichlorométhane puis un mélange dichlorométhane/acétate d'éthyle 1/1 en volume.

La fraction rouge orange est récupérée et séchée. On obtient 8,5 g d'un solide rouge (rendement 70 %).

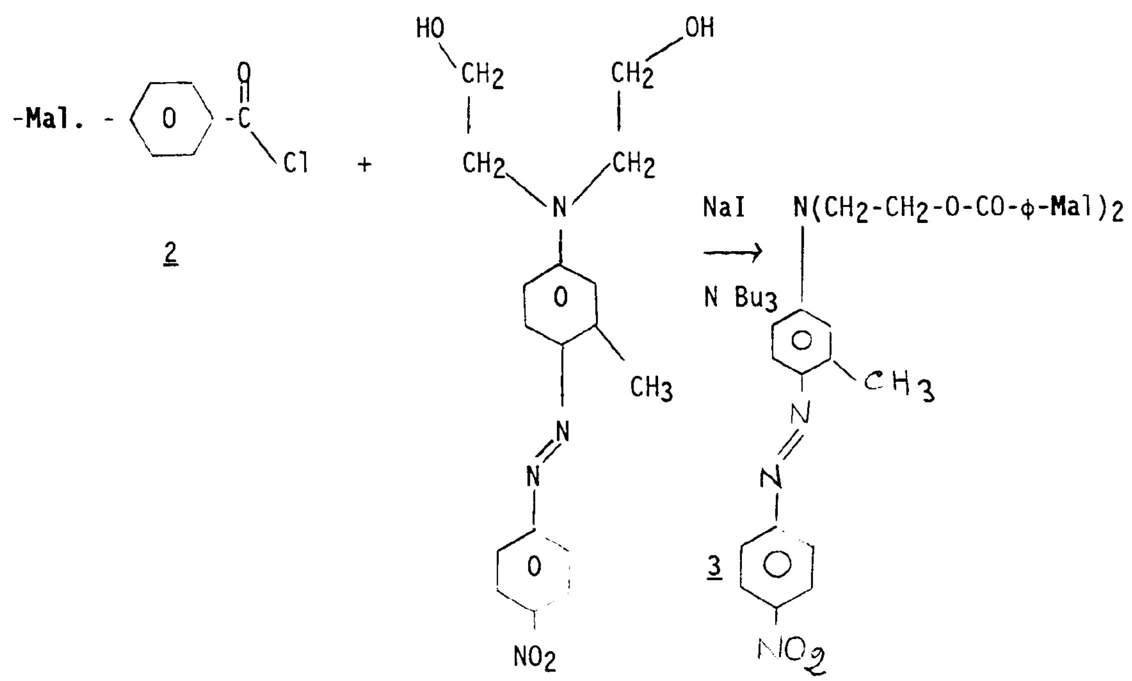


Les données spectroscopiques (RMN, Masse) confirment la structure proposée.

Exemple 2

Un mélange (85 % en masse N-vinyl pyrrolidone ; 15 % en masse molécules 1)

1

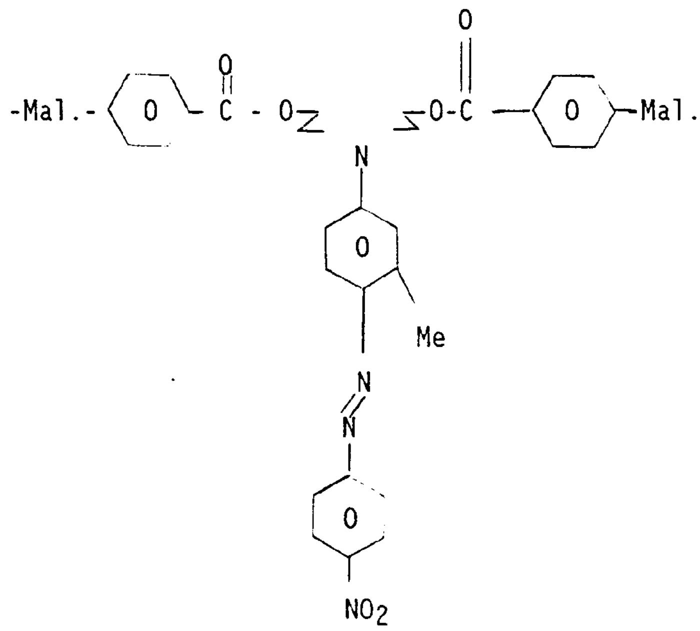

est chauffé à 120° C pendant un interval de temps variable (de 15 min à 120 min). Le degré d'avancement de polymérisation varie avec le temps de chauffage. La Tg des films, préparés au tire-film à partir des collodions est également modifiée. Les Tg, mesurées à 50Hz par la variation de la constante diélectique du film en fonction de la température, s'échelonnent entre 90° C et 150° C (pour un collodion 30 min à 120° C et 180 min à 120° 120° C) à T > Tg, on polarise les films obtenus en appliquant un champ continu qui est d'environ 20 V/μm. Après polarisation, on refroidit le film jusqu'à température ambiante en maintenant un champ tel que

$$\frac{E\ polarisation}{T} = constante$$

On réticule les films obtenus et on augmente ainsi la Tg par irradiation sous faisceau d'électrons [16 MRad (0,16 M grades) sous faisceau d'électrons en rideau]. Ainsi, on passe pour un film dont la Tg est de 140° C (prépolymérisation de 120 min à 120° C) avant radiation à 190°C après irradiation.

Ainsi, l'activité électro-ontique mesurée par une méthode PFOI, référence: R MEYRUEIX, G. MIGNANI, G. TAPOLSKY, SPIE vol. 1127, 160 (1989) de ces films polarisés et réticulés sous faisceau d'électrons est dans les limites de détection, constante et ce au-delà d'une période de 2 mois.

Exemple N°3

Synthèse :

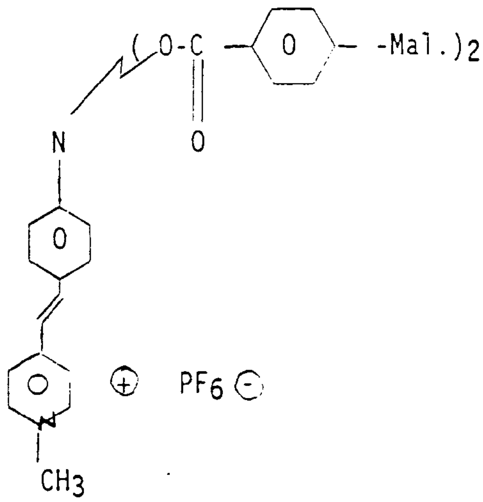

Dans un tricol de deux litres, on introduit

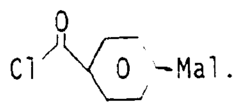

81,3 g (0,345 moles), 800 ml de diméthylacétamide anhydre (DMAC), 51,5g (0,116 mole) de

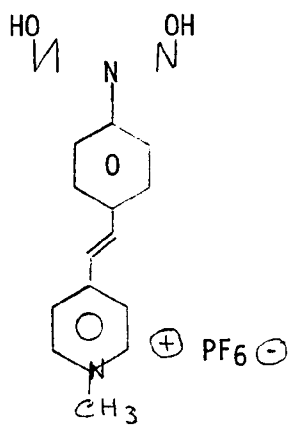

2,5 g (0,0167 mole) de NaI et 53 g de tributylamine, on laisse réagir 3 h à la température ambiante et 16 h à 40°C. On précipite dans 61 d'eau ; il y a formation d'un précipité orange ; on filtre, on rince abondamment à l'eau (pour éliminer l'acide

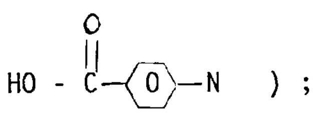

on sèche sous vide le précipité et on récupère 98 g d'un solide orange. A l'aide d'une chromatographie sur une colonne de gel de silice (éluant acétone/hexane//75/25//V/V) + 1 ml d'une solution $NH_4PF_6$, $H_2O$ par litre d'éluant.

On récupère 9 g d'un solide orange de température de fusion : (95° C (décomposition) : Rendement isolé : 10,2 %).

Les analyses IR, UV, $^1$HRMN et spectre de masse confirment la structure du produit.

Précipitation du collodion : filmature

Le collodion est préparé dans la NVP (N.Vinyl pyrolidone) dans une concentration massique : molécule active/NVP = 85/15. Ce collodion est chauffé à 120° C durant 1 h ; le collodion ainsi préparé est filtré sur 0,2μ.m puis filmé par les méthodes classiques (Tire film ; barre de Mayer ).

Polarisation et réticulation (voire identique à l'exemple 1)

Exemple 4

**Synthèse du polymère B.**

On s'inspire de l'article de B. Kovacic, J.Org. Chem. (1959), 1187. Dans un ballon de 250 ml de DMAC anhydre, on dissout 860 mg de du monomère A (1.168 mmol). A cette solution on ajoute une quantité stoechiométrique de N,N'-diéthyl-hexaméthylène-diamine. La solution est portée à 110°C sous azote pendant 48 heures. Une fois la température de la solution revenue à température ambiante, on précipite dans l'eau. On récupère un précipité rouge qu'on lave à l'eau. Il est ensuite séché sous vide pendant 12 heures. On obtient 900 mg de polymère dont la température de transition vitreuse commençante (**Differential-scanning calorimetry Perkin-Elmer DSC7**) est de 77 ± 2°C et dont la structure, illustrée ci-dessous, est confirmée par les analyses spectroscopiques RMN, IR.

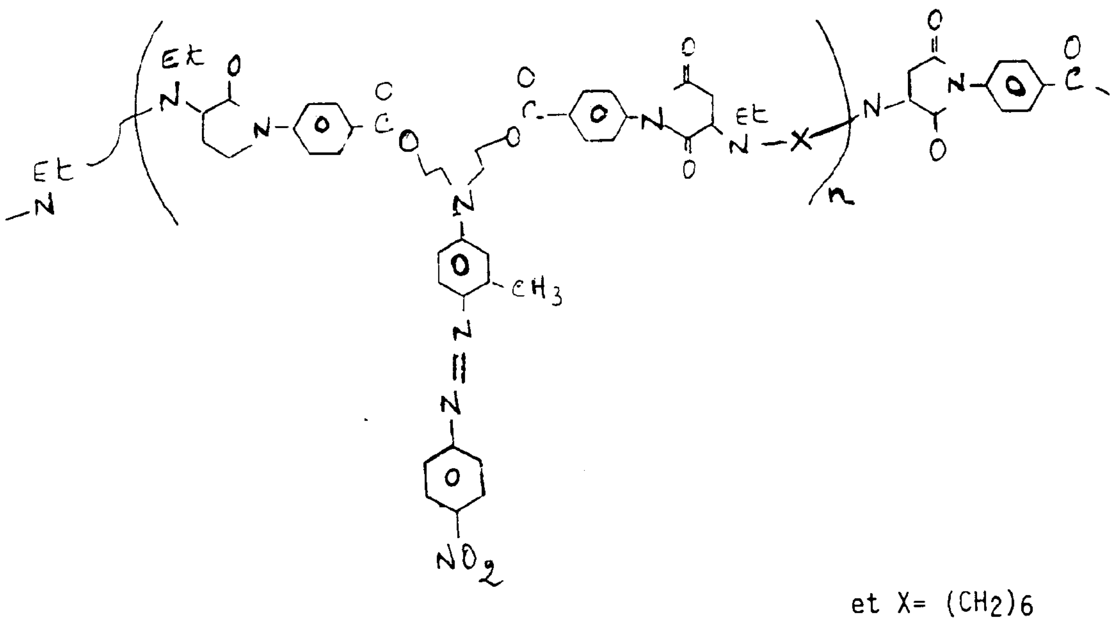

et X= $(CH_2)_6$

La température de transition vitreuse du polymère B peut être modifiée par l'utilisation de diamines différentes et aromatiques ainsi que cela est décrit dans les exemples 5 et 6.

Exemple 5

**Synthèse du polymère C.**

Selon la méthode décrite dans l'exemple 4, on dissout 517 mg (0,7 mmol) de A dans 20 ml de DMAC et l'on ajoute 43 mg (0,7 mmol) d'éthylène diamine. Après 48 heures à 110°C, on précipite le polymère dans le méthanol et on sèche le précipité sous vide. La température de transition vitreuse de ce polymère est de 107° ±2°C, sa structure illustrée ci-dessous, est confirmée par RMN et IR.

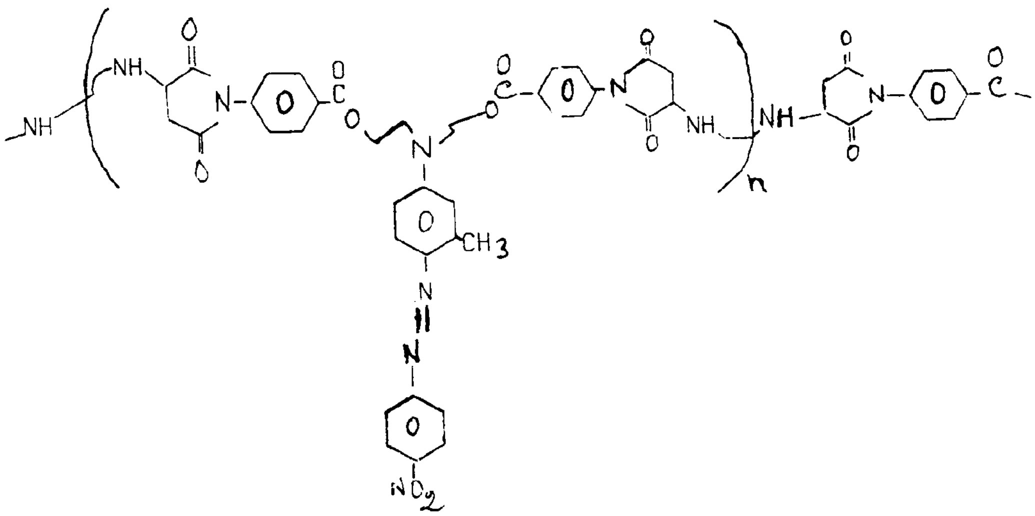

Exemple 6

**Synthèse du polymère D.**

Synthèse identique aux précédentes mais cette fois on utilise la para-phénylène-diamine. Dans 25 ml de DMAC, on dissout 0,8 mmol de p-phénylène diamine. On ajoute 25 mg de **peroxyde de benzoyle** comme catalyseur de polymérisation. Après 24 heures à 110°C, on précipite dans l'eau et on obtient un précipité rouge dont la structure -cf. ci-dessous-est confirmée par RMN et IR et dont la température de transition vitreuse est de 125°C ±2°C.

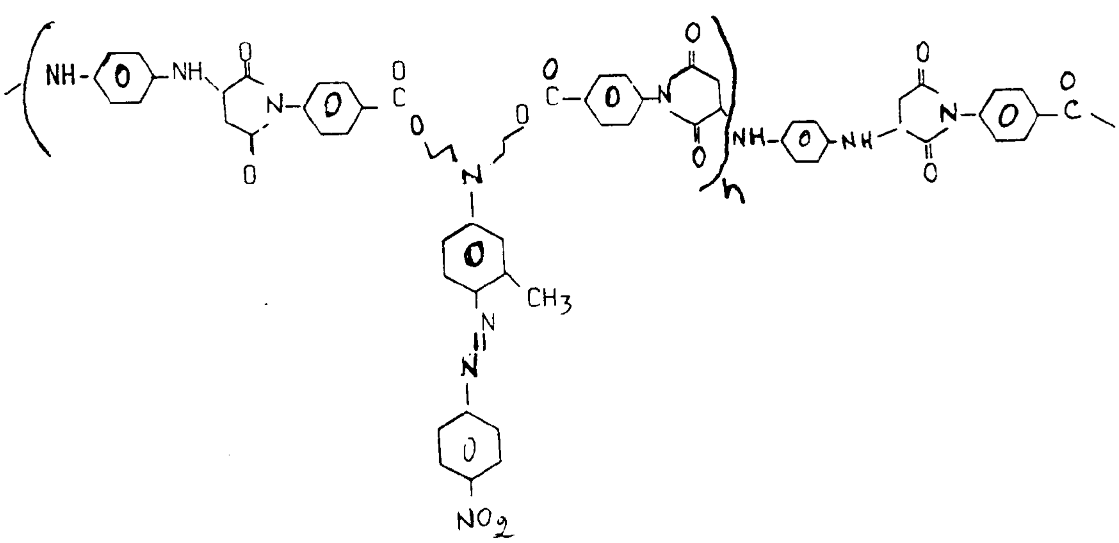

Exemple 7

**Synthèse du polymère E.**

On s'inspire de l'article R. Bacskaj J. Pol. Sci., A (10), 1297, 1972. On dissout 736 mg (1 mmol) de monomère A dans du THF fraichement distillé. On ajoute 2 équivalents d'éthyl-vinyl éther soit 145 mg et 50 mg de peroxyde de benzoyle. On porte la solution à reflux sous azote. Après 24 heures, on laisse la solution revenir à température ambiante puis on précipite dans le méthanol. On récupère un précipité rouge qui est lavé au méthanol puis séché sous vide. La structure du polymère décrite ci-dessous est confirmée par analyses spectroscopiques RMN et IR. La temperature de transition vitreuse est de 120 ±2°C.

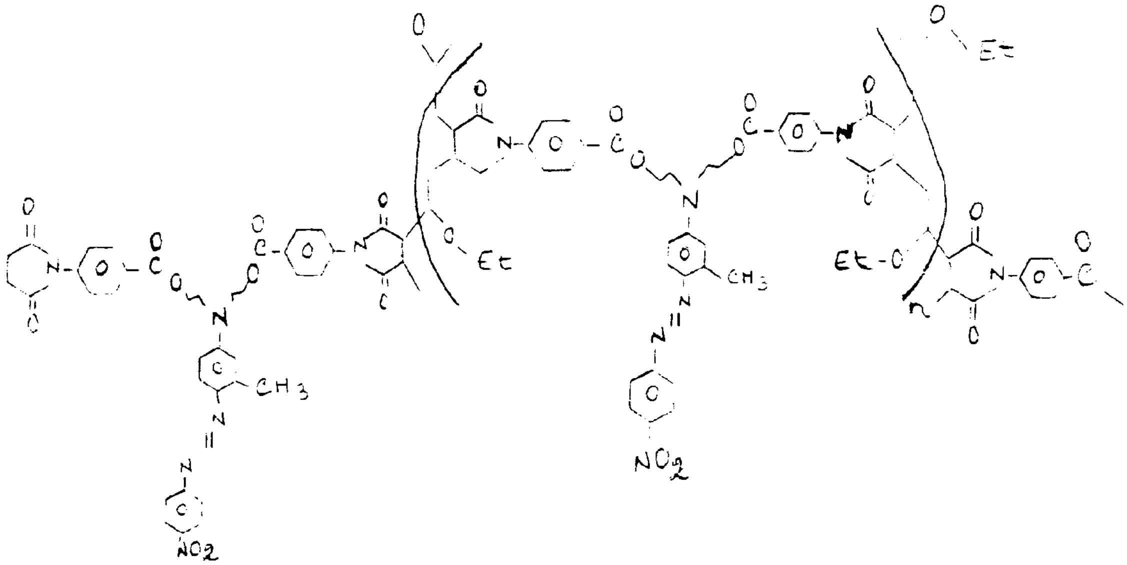

Comme il a été décrit dans l'exemple 2, il est possible de polariser les films obtenus et de les post réticuler par les méthodes bien connues de l'homme de l'art comme par exemple la photoréticulation, la réticulation sous faisceau d'électrons ou la thermoréticulation.

Exemple 8

**Synthèse du polymère F.**

Synthèse identique à celle décrite dans l'exemple 5 mais avec 0,7 mmol de monomère A et 0,45 mmol d'éthylène diamine. Le polymère obtenu a une température de transition vitreuse de 103° ±2°C. On peut filmer une solution de ce polymère dans la DMAC par les méthodes habituelles décrites préalablement. Le film est ensuite réticulé sous faisceau d'électrons selon la méthode décrite dans l'exemple 2. La température de transition vitreuse du film est après réticulation de 140 ± 2°C. On peut également tirer un film à partir d'une solution de ce polymère contenant de la N-vynylpyrrolidone et irradier le film obtenu. La température de transition vitreuse alors obtenue est de 147 ± 2°C.

Exemple 9

**Synthèse du polymère G.**

Synthèse identique à l'exemple 7 mais dans un rapport molaire différent. Pour 736 mg du monomère A, on ajoute 0,65 mmol d'éthylvinyl -éther. Le polymère obtenu a une température de transition vitreuse de 109° + 2°C. On peut filmer une solution de ce polymère dans la DMAC contenant de la N-vinyl-pyrrolidone. Il est possible de réticuler ce film sous faisceau d'électrons selon la méthode décrite dans l'exemple 2. La température de transition vitreuse du film est alors de 175°C.

On peut réticuler les films sous faisceau d'électrons ainsi que cela a été décrit dans les exemples 8 et 9. Il est aussi possible de photoréticuler ces mêmes films à l'aide de photo**initiateurs** appropriés e+ décrits dans la littérature.

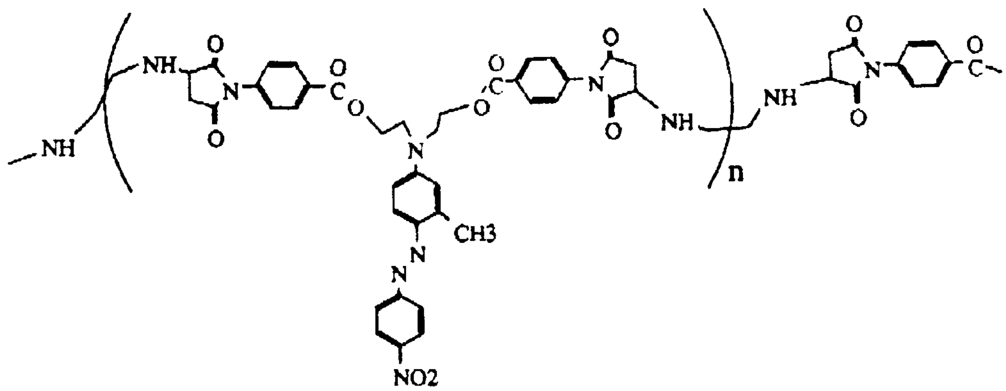

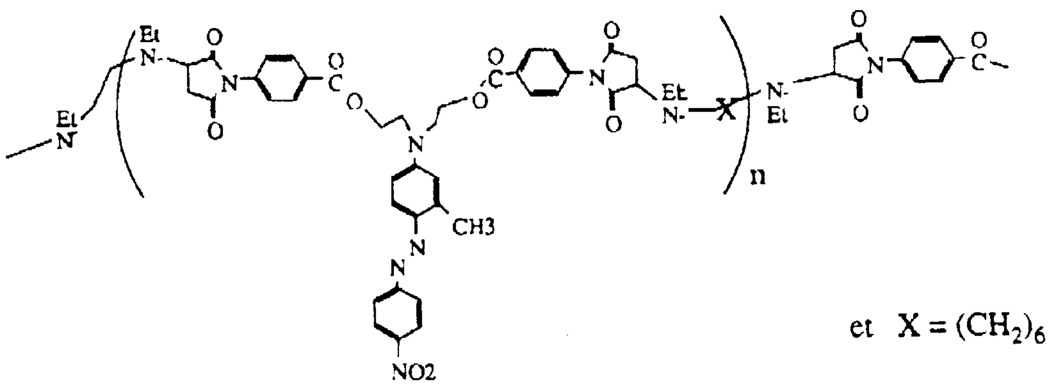

et $X = (CH_2)_6$

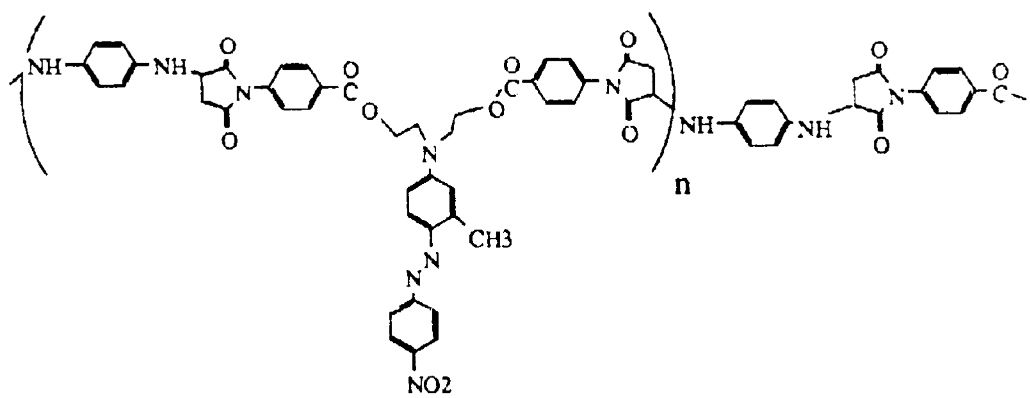

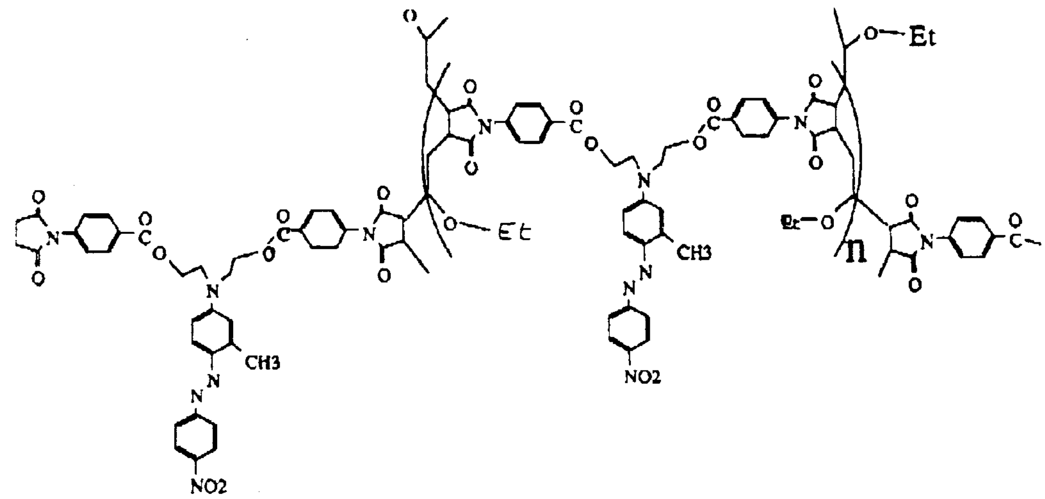

Structure que l'on peut représenter également par :

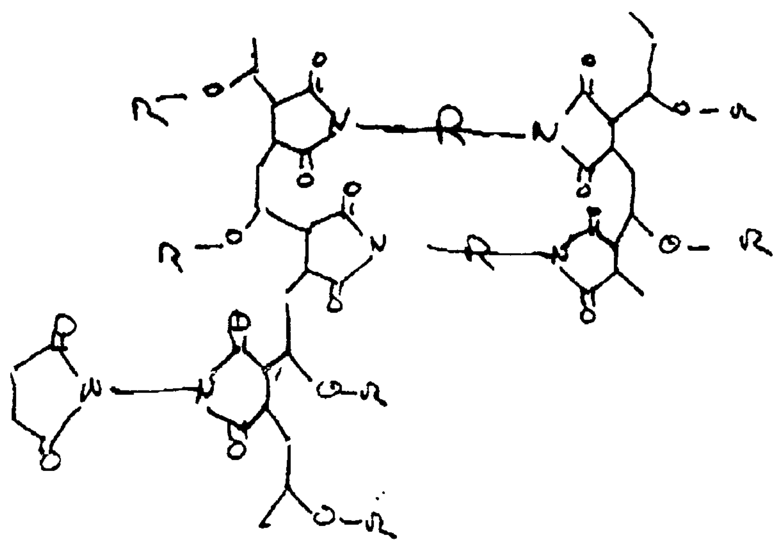

<u>Exemple 10</u>

**Synthèse du polymère H.**

Synthèse identique à celle décrite dans l'exemple 6 mais avec 0,7 mmol de monomère A et 0,45 mmol de para-phénylène diamine. Le polymère obtenu a une température de transition vitreuse de 113°C ± 2°C. On peut filmer une solution de ce polymère dans la DMAC par les méthodes habituelles décrites préalablement. Il est ensuite possible de réticuler un film sous faisceau d'électrons selon la méthode décrite dans l'exemple 2.

Il est également possible de réticuler thermiquement ces films. Un film obtenu à partir du collodion obtenu et contenant de la N-vynylpyrrolidone (10 % massique) est filmé. Le film est polarisé et simultanément réticulé thermiquement, tel que nous l'avons décrit dans l'étape de polarisation, pour obtenir une température de transition vitreuse d'au moins 180°C.

**Revendications**

**1)** Composé caractérisé par le fait que il comporte :

– au moins deux doubles liaisons carbone-carbone, chaque double liaison étant activée, c'est-à-dire dont la polymérisation est facilitée, par une fonction électroattractrice ;

– un système non centro symétrique de liaisons pi conjugées portant à l'une des extrémités au moins un groupe électroattracteur et à l'autre extrémité au moins un groupe électrodonneur.

**2)** Composé selon la revendication 1 caractérisé par le fait qu'il présente la formule suivante :

```
   Y'                                        Y'
   !                                         !
R-C = CH-CO-N (R') A - N (R') - CO-CH = CR            (I)
```

dans laquelle :

les radicaux R représentent indépendamment un atome d'hydrogène ou des radicaux alcoyle ou alcoylidène de bas poids moléculaire (de $C_1$ à $C_6$) éventuellement substitué.

R' représente indépendamment un groupe acyle de $C_1$ à $C_6$ éventuellement substitué ou un groupe acylidène de $C_1$ à $C_6$, éventuellement substitué et/ou insaturé.

R et R' étant avantageusement reliés entre eux pour former un hétérocycle de 5 à 7 chainons ;

Où A représente le système à liaison $\pi$ équipé de deux liens, différents ou de préférence semblable, L et L' tel que A puisse être représente par

-L-A'-L'-

avec A' représentant le système à liaison pi.

**3)** Composé selon l'une des revendications 1 et 2 caractérisé par le fait les radicaux R et R' sont choisis de manière que la formule (I) devienne la formule (I')

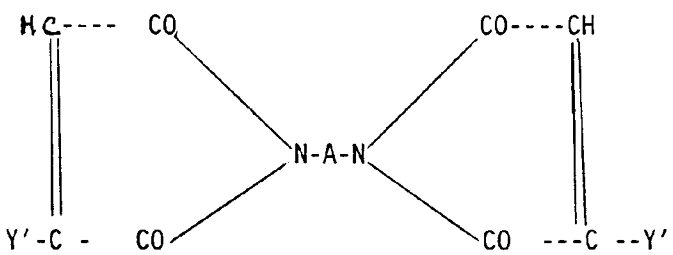

(I')

dans laquelle :

. les symboles Y' représentent indépendamment H, $CH_3$ ou Cl ;

**4)** Composé selon l'une des revendications 1 à 3 caractérisé par le fait que A' représente un groupement hyperpolarisable de formule :

$$E-Z_1-X = Y-Z_2-N$$

dans laquelle :

– X et Y représentent indépendamment N, C et/ou CH

– $Z_1$, $Z_2$ représentent un groupe aromatique ou insaturé divalent choisi par exemple parmi ceux dérivés le benzène (c'est-à-dire phénylène), la pyridine, le furanne, le styrène, l'acétylène, l'éthylène.

– E représente un groupe électroaccepteur.

**5)**(Co) polymère caractérisé par le fait qu'il comporte au moins 10 % en mole de motif issu d'un monomère selon la revendication 1.

**6)** Polymère selon la revendication 5 caractérisé par le fait qu'il est polarisé et présente un <cos θ> compris entre $10^{-2}$ et 0,5.

**7)** Matériau caractérisé par le fait qu'il comporte un polymère selon la revendication 5.

**8)** Film caractérisé par le fait qu'il est constitué en matériau selon la revendication 7.

**9)** Dispositif en optique caractérisé par le fait qu'il comporte un élément réalisé en matériau selon la revendication 2.

**10)** Procédé de synthèse d'un polymère comportant des motifs actifs en ONL, caractérisé par le fait que

l'on soumet un composé monomère selon la revendication 1 soit on mélange avec d'autres monomères à une polymérisation par chauffage à une température comprise entre 50 et 200°C.

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande

EP 91 42 0345

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | JOURNAL OF POLYMER SCIENCE: PART A: POLYMER CHEMISTRY, vol. 27, 1989, pages 3465-3479, John Wiley & Sons, Inc.; J. A. MIKROYANNIDIS: "New heat-resistant polymers derived from azomethine bismaleimides and maleimide-terminated azomethine prepolymers"<br>* Composés 1-4 du tableau 1 *<br>--- | 1-4 | C 07 D 207/452<br>C 07 D 207/456<br>C 08 F 226/10<br>C 08 J 5/18<br>G 02 F 1/35 |
| D,A | US-A-4 757 130 (DeMARTINO)<br>* En entier *<br>----- | 1-4 | |
| | | | **DOMAINES TECHNIQUES RECHERCHES (Int. Cl.5)** |
| | | | C 07 D 207/00 |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes:

Revendications ayant fait l'objet de recherches incomplètes: 1-10

Revendications n'ayant pas fait l'objet de recherches:

Raison pour la limitation de la recherche:

Les revendications 1-4 concernant composes de formules I et I' sont trop vagues pour effectuer une recherche complete. Aussi il manque une definition pour les variables L, L' et E. Seulement des composes ont fait object de la recherche qui sont characterisés par le groupement A= E-Z1-X=z-Z2-N (X,Y=N,C; Z1,Z2=noyeau aromatique) correspondant a la formule I'.

| Lieu de la recherche | Date d'achèvement de la recherche | Examinateur |
|---|---|---|
| LA HAYE | 19-11-1991 | KISSLER B.E. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0408)